(19) Europäisches Patentamt
European Patent Office
Office européen des brevets

(11) EP 2 787 965 B1

(12) **EUROPEAN PATENT SPECIFICATION**

(45) Date of publication and mention of the grant of the patent:
**12.06.2019 Bulletin 2019/24**

(21) Application number: **12798281.7**

(22) Date of filing: **06.12.2012**

(51) Int Cl.:
***A61K 8/92*** *(2006.01)* ***A61K 8/9789*** *(2017.01)*
***A61Q 5/06*** *(2006.01)* ***A61K 8/02*** *(2006.01)*

(86) International application number:
**PCT/EP2012/074657**

(87) International publication number:
**WO 2013/083701 (13.06.2013 Gazette 2013/24)**

(54) **COMPOSITION BASED ON RED HENNA POWDER AND OIL(S), AND HAIR DYEING PROCESS USING THIS COMPOSITION**

ZUSAMMENSETZUNG AUF DER BASIS VON ROTEM HENNAPULVER UND ÖL(EN) SOWIE HAARFÄRBEVERFAHREN MIT DIESER ZUSAMMENSETZUNG

COMPOSITION À BASE DE POUDRE DE HENNÉ ROUGE ET D'UNE OU PLUSIEURS HUILES ET PROCÉDÉ DE COLORATION DE CHEVEUX UTILISANT CETTE COMPOSITION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2011 FR 1161385**
**29.12.2011 US 201161581174 P**

(43) Date of publication of application:
**15.10.2014 Bulletin 2014/42**

(73) Proprietor: **L'Oréal**
**75008 Paris (FR)**

(72) Inventor: **POURILLE, Chrystel**
**F-95110 Sannois (FR)**

(74) Representative: **Rivière, François Armand**
**L'Oréal**
**Service DIPI**
**9 Rue Pierre Dreyfus**
**92110 Clichy (FR)**

(56) References cited:
**WO-A1-02/47634** **DE-A1- 3 609 962**
**DE-A1- 19 600 225** **DE-A1- 19 905 707**
**JP-A- 2002 284 653**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The invention relates to a composition A, which is preferably compact and/or anhydrous, comprising at least 20% of red henna powder (*Lawsonia inermis, alba*) and at least one oil chosen from babassu oil, sunflower oil, olive oil, coconut oil, Brazil nut oil, marula oil, corn oil, argan oil, soybean oil, grapeseed oil, flax oil, sesame oil, hazelnut oil, apricot oil, macadamia oil, arara oil, castor oil, avocado oil, shea butter oil, coprah oil and rapeseed oil, in which the oil(s) are in an amount inclusively between 1% and 80% by weight relative to the total weight of the composition and also an aqueous composition B derived from A for dyeing keratin fibres, to the process for dyeing keratin fibres using composition A or B, and to the use of compositions A or B for dyeing keratin fibres.

Two major methods for dyeing human keratin fibres, and in particular the hair, are known.

The first, known as oxidation dyeing or permanent dyeing, consists in using one or more oxidation dye precursors, more particularly one or more oxidation bases optionally combined with one or more couplers.

Oxidation bases are usually selected from ortho- or para-phenylenediamines, ortho- or para-aminophenols, and heterocyclic compounds. These oxidation bases are colourless or weakly coloured compounds, which, when combined with oxidizing products, can give rise via a process of oxidative condensation to coloured species, which remain trapped within the fibre.

The shades obtained with these oxidation bases are often varied by combining them with one or more couplers, these couplers being chosen especially from aromatic *meta*-diamines, *meta*-aminophenols, *meta*-diphenols and certain heterocyclic compounds, such as indole compounds.

The variety of molecules used as oxidation bases and couplers allows a wide range of colours to be obtained.

The second dyeing method, known as direct dyeing or semipermanent dyeing, comprises the application of direct dyes, which are coloured and colouring molecules that have affinity for fibres. Given the nature of the molecules used, they tend rather to remain on the surface of the fibre and penetrate relatively little into the fibre, when compared with the small molecules of oxidation dye precursors. The main advantages of this type of dyeing are that it does not require any oxidizing agent, which limits the degradation of the fibres, and that it does not use any dyes that have particular reactivity, resulting in limitation of the intolerance risks.

The first hair dyes were semi-permanent. One of the most well known natural dyes is that derived from the henna plant. Henna is still be used in feminine beauty enhancement for colouring the hair or the nails, or for dyeing leather, silk and wool, etc. It is also be used traditionally for various important events, celebrations and beliefs.

Red henna consists of leaves of shrubs of the genus *Lawsonia* from the family of *Lythraceae,* and is based on the principle of dyeing with the active agent lawsone: 2-hydroxy-1,4-naphthoquinone. Lawsone [83-72-7] (CI Natural Orange 6; CI 75420), also known as isojuglone, may be found in henna shrubs (*Lawsonia alba, Lawsonia inermis*) ("Dyes, Natural", Kirk-Othmer Encyclopedia of Chemical Technology*, "*Henna" Encyclopedia Britannica*)* .

This dye affords an orange-red coloration on grey hair, and a "warm" i.e. coppery to red colour on chestnut-brown hair. The dyeing process using henna is difficult to perform. A kind of "paste" (often referred to as a "poultice") is first made from ground or powdered henna leaves, which is then diluted at the time of use with warm water, and the said paste is then applied to the keratin fibres.

However, this process using the said paste has drawbacks. During the preparation and application of the composition to keratin fibres, it is not always possible to obtain satisfactory impregnation due to the poor consistency of the composition obtained from the coarsely ground powder. Furthermore, it is very difficult to aim to reproduce shades exactly, since the lawsone content very often varies from one batch to another and between different ground materials.

The handling of the powder may result in it becoming spread around the surrounding area and lead to irritation problems.

Added to this are the risks of staining of clothing and the skin during the preparation of the "paste" and also during its application to the keratin fibres, since the consistency is very irregular.

In addition, the leave-on time is long. It may vary from several tens of minutes to several hours (overnight) depending on the desired intensity, with no ability to control the result. The result varies as a function of the fibres to be dyed and of the henna raw material used.

As much as the colour obtained on chestnut-brown hair has a natural look, grey hair is dyed an unaesthetic and unnatural orange colour ("Hair preparations", Kirk-Othmer Encyclopedia of Chemical Technology, John Wiley & Sons, Inc.). Furthermore, the colorations obtained are not uniform between the root and the end or from one fibre to another (The Science of Hair Care, C. Bouillon, J. Wilkinson, 2nd Ed., CRC Press, Taylor & Francis Group; Boca Raton, London, pp. 236-241 (2005)).

It is known practice to use metal salts as mordants for improving the coloration of henna (Ullmann's Encyclopedia, 2006 Wiley-VCH Verlag GmbH & Co. KGaA, Weinheim 10.1002/14356007.a12 571.pub2 and US 2010/03133362). The use of these agents requires great know-how, multiplies the steps of the process, is not always friendly towards the integrity of the fibre (cosmetically unfriendly) and may disrupt subsequent cosmetic treatments.

Another solution for improving the coloration of henna is to use very finely ground henna leaf powder, optionally

in the presence of excipients in powder form (DE 299 02 432). Generally, these powders are conditioned in sachets or blister packs. If the conditioning of the henna powder happens to become broken, a large quantity of raw material is lost into the air and cannot be recovered. Added to the problem of the loss of raw material is that linked to the pulverulence for users as indicated previously. Specifically, in the form of fine particles, henna becomes suspended in the atmosphere and can cause respiratory problems or allergies such as rhinitis for the users, sellers of extracts or natural dyeing professionals (Allergy, J. Scibilia, E. Galdi, G. Biscaldi, G. Moscato, 52, 231-232, (1997)).

To overcome the problem of the poor dyeing efficacy of henna, it is known practice to "dope" coloration by adding direct dyes that are generally used in direct dyeing, such as nitrobenzene, anthraquinone, nitropyridine, azo, methine, azomethine, xanthene, acridine, azine or triarylmethane direct dyes (DE 199 05 707, EP 0 806 199, JP 2010-0001278). This option has the drawback for natural product users, or for partisans of "natural/organic" products, in that the coloration is partly performed using synthetic dyes. International patent application WO 97/39724 describes hair treatment compositions in solid form and compounds whose viscosity does not exceed 500 mPa.s. The proposed compositions cannot produce rapid or satisfactory colorations. Furthermore, the solid compositions do not always readily break down in water. in addition, the poultices derived from the compositions of the prior art are not always creamy and/or easy to apply.

There is thus a real need to develop dyeing processes that can produce powerful colorations using henna, while at the same time being friendly to the cosmetics of keratin fibres. In particular, there is a need to provide a henna-based dyeing product that does not have the drawbacks mentioned above, especially a dust-free product that is easy to store, readily miscible (rapid breakdown) in water, and that can especially produce colorations that are less aggressive to the hair and at the same time that are resistant to external agents (light, bad weather or shampooing), and that are fast and homogeneous, while at the same time remaining powerful and chromatic. Moreover the colour uptake on the keratin fibers, especially hair treated with henna must be satisfactory.

This aim is achieved by the present invention, one subject of which is a composition in compact or non compact form, preferably in compact form and/or anhydrous form, comprising:

i) at least 20% by weight, relative to the weight of the composition, of red henna powder, preferably as fine particles, and

ii) at least one oil chosen from babassu oil, sunflower oil, olive oil, coconut oil, Brazil nut oil, marula oil, corn oil, argan oil, soybean oil, grapeseed oil, flax oil, sesame oil, hazelnut oil, apricot oil, macadamia oil, arara oil, castor oil, avocado oil, shea butter oil, coprah oil and rapeseed oil.;

in which the oil(s) are in an amount inclusively between 1 % and 80% by weight relative to the total weight of the composition.

Another subject of the invention is the aqueous composition (composition B) derived from the mixture between the compact and/or anhydrous composition (composition A) with water. This composition in particular, which is usually in the form of a poultice, is prepared from composition A defined above and an
water, in proportions ranging from 1 part by weight of composition per 1 part by weight of water (1/1) to 1 part by weight of composition per 3 parts by weight of an aqueous water (1/3), preferentially 1 part by weight of composition per 2 parts by weight of water (1/2).

A subject of the invention is also a process for dyeing keratin fibres, especially the hair, using compositions A or B, and the use of these compositions for dyeing keratin fibres, especially the hair.

The composition and poultice according to the invention have the advantage of dyeing keratin fibres, especially human keratin fibres, with strong, chromatic colorations that are resistant to washing, perspiration, sebum and light, and that are moreover long-lasting, without impairing the said fibres. Furthermore, the colorations obtained using the composition or the poultice give uniform colours from the root to the end of a fibre (little coloration selectivity). Furthermore, the application of the composition or of the poultice does not give off any raw material dust (dust-free). The composition or the poultice is easy to use, in total safety and with no risk of staining. In addition, the composition and the active agent remain stable on storage. The treated keratin fibres have a very pleasant cosmetic aspect, their integrity is respected.

In addition, the composition of the invention, even in compact form, is very water-miscible even in cold water (especially between 10°C and room temperature, 25°C), and the poultice then formed is particularly creamy and/or shows excellent adhesion to the hair. Moreover, the time and/or ease of breakdown of the composition when it is in compact and preferably anhydrous form is faster or easier, for an equivalent amount, than the compact compositions on the market.

*i) Red henna powder*

The composition according to the invention comprises as first ingredient red henna in powder form.

The henna powder may be screened to obtain particles with upper limit sizes corresponding to the orifices or

mesh sizes of the screen particularly between 35 and 80 mesh (US).

According to one particular mode of the invention, the size of the henna powder particles is fine. According to the invention, a particle size of less than or equal to 500 μm is more particularly intended. Preferentially, the powder consists of fine particles with sizes inclusively between 50 and 300 μm and more particularly between 10 and 200 μm.

It is understood that the said henna particles preferentially have a moisture content of between 0 and 10% by weight relative to the total weight of the powders.

Composition A according to the invention comprises red henna powder in an amount particularly inclusively between 20% and 99% by weight, relative to the total weight of the composition, more particularly between 30% and 95% by weight, preferentially between 40% and 90% by weight, more preferentially between 50% and 85% by weight and even more preferentially between 60% and 80% by weight.

*ii) Oils*

The composition of the invention comprises as second ingredient one or more identical or different oils.

The term "*oil*" means a "*fatty substance*" that is liquid at ambient temperature (25°C) and at atmospheric pressure (760 mmHg). The viscosity at 25°C is preferably less than 1200 cps and better still less than 500 cps (defined, for example, from the Newtonian plateau determined using an ARG2 rheometer from TA Instruments equipped with a spindle with cone-plate geometry 60 mm in diameter and with an angle of 2 degrees over a shear stress range of from 0.1 Pa to 100 Pa).

The term "*fatty substance*" means an organic compound that is insoluble in water at ordinary temperature (25°C) and at atmospheric pressure (760 mmHg) (solubility of less than 5%, preferably of less than 1% and more preferably still of less than 0.1%). They have in their structure at least one hydrocarbon chain comprising at least 6 carbon atoms or a sequence of at least two siloxane groups. In addition, the fatty substances are generally soluble in organic solvents under the same temperature and pressure conditions, for instance chloroform, dichloromethane, carbon tetrachloride, ethanol, benzene, toluene, tetrahydrofuran (THF), liquid petroleum jelly or decamethylcyclopentasiloxane.

According to the invention, the oils are chosen from babassu oil, sunflower oil, olive oil, coconut oil, Brazil nut oil, marula oil, corn oil, argan oil, soybean oil, grapeseed oil, flax oil, sesame oil, hazelnut oil, apricot oil, macadamia oil, arara oil, castor oil, avocado oil, shea butter oil and rapeseed oil.

More particularly, the oils of plant origin are chosen from avocado oil, olive oil, coconut oil, copra oil, argan oil and sunflower oil.

Composition A according to the invention comprises one or more oils in an amount inclusively between 1% and 80% by weight, relative to the total weight of the composition, more particularly inclusively between 2% and 50% by weight, preferentially inclusively between 3% and 40% by weight and more preferentially inclusively between 5% and 25% by weight.

*iii) Optionally at least one fatty substance other than oils: butters, waxes or resins*

The composition of the invention may also comprise one or more fatty substances other than the oil(s) as defined previously.

According to one particular embodiment of the invention, the composition comprises as third constituent one or more butters, preferably of plant origin.

For the purposes of the present invention, the term "butter" (also known as a "pasty fatty substance") means a lipophilic fatty compound which undergoes a reversible solid/liquid change of state and which comprises, at a temperature of 25°C and at atmospheric pressure (760 mmHg), a liquid fraction and a solid fraction. In other words, the starting melting point of the pasty compound may be less than 25°C. The liquid fraction of the pasty compound, measured at 25°C, may represent 9% to 97% by weight of the compound. This fraction that is liquid at 25°C preferably represents between 15% and 85% and more preferably between 40% and 85% by weight.

Preferably, the butter(s) have an end melting point of less than 60°C.

Preferably, the butter(s) have a hardness of less than or equal to 6 MPa.

Preferably, the pasty fatty substances have, in the solid state, an anisotropic crystal organization, which is visible by X-ray observation.

For the purpose of the invention, the melting point corresponds to the temperature of the most endothermic peak observed on thermal analysis (DSC) as described in Standard ISO 11357-3; 1999. The melting point of a pasty substance or of a wax may be measured using a differential scanning calorimeter (DSC), for example the calorimeter sold under the name DSC Q2000 by the company TA Instruments.

As regards the measurement of the melting point and the determination of the end melting point, the sample preparation and measurement protocols are as follows:

A sample of 5 mg of pasty fatty substance, preheated to 80°C and withdrawn with magnetic stirring using a spatula that

is also heated, is placed in a hermetic aluminium capsule, or a crucible. Two tests are performed to ensure the reproducibility of the results.

The measurements are performed on the abovementioned calorimeter. The oven is flushed with nitrogen. Cooling is performed by an RCS 90 heat exchanger. The sample is then subjected to the following protocol: it is first placed at a temperature of 20°C, and then subjected to a first temperature rise passing from 20°C to 80°C, at a heating rate of 5°C/minute, then is cooled from 80°C to -80°C at a cooling rate of 5°C/minute and finally subjected to a second temperature rise passing from -80°C to 80°C at a heating rate of 5°C/minute. During the second temperature increase, the variation of the difference in power absorbed by the empty crucible and by the crucible containing the sample of butter is measured as a function of the temperature. The melting point of the compound is the value of the temperature corresponding to the top of the peak of the curve representing the variation in the difference in power absorbed as a function of the temperature.

The end melting point corresponds to the temperature at which 95% of the sample has melted.

The liquid fraction by weight of the butter at 25°C is equal to the ratio of the enthalpy of fusion consumed at 25°C to the enthalpy of fusion of the butter.

The enthalpy of fusion of the pasty compound is the heat consumed by the compound in order to pass from the solid state to the liquid state. The butter is said to be in the solid state when all of its mass is in crystalline solid form. The butter is said to be in the liquid state when all of its mass is in liquid form.

The enthalpy of fusion of the butter is equal to the integral of the entire melting curve obtained using the abovementioned calorimeter, with a temperature rise of 5 or 10°C/minute, according to standard ISO 11357-3:1999. The enthalpy of fusion of the butter is the amount of energy required to make the compound change from the solid state to the liquid state. It is expressed in J/g.

The enthalpy of fusion consumed at 25°C is the amount of energy absorbed by the sample to change from the solid state to the state that it has at 25°C, composed of a liquid fraction and a solid fraction.

The liquid fraction of the butter measured at 32°C preferably represents from 30% to 100% by weight of the compound, preferably from 50% to 100%, more preferably from 60% to 100% by weight of the compound. When the liquid fraction of the butter measured at 32°C is equal to 100%, the temperature of the end of the melting range of the pasty compound is less than or equal to 32°C.

The liquid fraction of the butter measured at 32°C is equal to the ratio of the enthalpy of fusion consumed at 32°C to the enthalpy of fusion of the pasty compound. The enthalpy of fusion consumed at 32°C is calculated in the same way as the enthalpy of fusion consumed at 23°C.

As regards the measurement of the hardness, the sample preparation and measurement protocols are as follows:

The composition according to the invention or the butter is placed in a mould 75 mm in diameter, which is filled to about 75% of its height. In order to overcome the thermal history and to control the crystallization, the mould is placed in a Vötsch VC 0018 programmable oven, where it is first placed at a temperature of 80°C for 60 minutes, then cooled from 80°C to 0°C at a cooling rate of 5°C/minute, and then left at the stabilized temperature of 0°C for 60 minutes, and then subjected to a temperature rise ranging from 0°C to 20°C at a heating rate of 5°C/minute, and then left at the stabilized temperature of 20°C for 180 minutes.

The compression force measurement is taken using a TA/TX2i texturometer from Swantech. The spindle used is chosen according to the texture:

- cylindrical steel spindle 2 mm in diameter for very rigid starting materials;
- cylindrical steel spindle 12 mm in diameter for sparingly rigid starting materials.

The measurement comprises three steps:

- a first step after automatic detection of the surface of the sample, where the spindle moves at a measuring speed of 0.1 mm/second, and penetrates into the composition according to the invention or the butter to a penetration depth of 0.3 mm, and the software notes the maximum force value reached;
- a second step, known as relaxation, where the spindle remains in this position for one second and the force is noted after 1 second of relaxation; and finally
- a third step, known as withdrawal, where the spindle returns to its original position at a speed of 1 mm/second, and the withdrawal energy of the probe (negative force) is noted.

The hardness value measured during the first step corresponds to the maximum compression force measured in newtons divided by the area of the texturometer cylinder expressed in $mm^2$ in contact with the butter or the composition according to the invention. The hardness value obtained is expressed in megapascals or MPa.

According to one preferred mode of the invention, the particular butter(s) are of plant origin, such as those

described in Ullmann's Encyclopedia of Industrial Chemistry ("Fats and Fatty Oils", A. Thomas, Published Online: 15 JUN 2000, DOI: 10.1002/14356007.a10_173, point 13.2.2.2. Shea Butter, Borneo Tallow, and Related Fats (Vegetable Butters)).

Mention may be made more particularly of shea butter, Karite Nilotica butter *(Butyrospermum parkii),* galam butter, (*Butyrospermum parkii),* Borneo butter or fat or tengkawang tallow (*Shorea stenoptera*), shorea butter, illipe butter, madhuca butter or *Bassia madhuca longifolia* butter, mowrah butter (*Madhuca latifolia*), katiau butter (*Madhuca mottleyana*), phulwara butter (*M. butyracea*), mango butter (*Mangifera indica*), murumuru butter (*Astrocaryum murumuru*), kokum butter (*Garcinia indica*), ucuuba butter (*Virola sebifera*), tucuma butter, painya butter (Kpangnan) (*Pentadesma butyracea*), coffee butter *(Coffea arabica),* apricot butter (*Prunus armeniaca*), macadamia butter (*Macadamia ternifolia*), grapeseed butter (*Vitis vinifera*), avocado butter *(Persea gratissima),* olive butter (*Olea europaea),* sweet almond butter *(Prunus amygdalus dulcis),* cocoa butter (*Theobroma* cacao) and sunflower butter.

According to one preferred mode of the invention, the weight content of $C_{16}$ fatty acid triglycerides, expressed relative to the total amount of fatty acid triglycerides in the butter(s) according to the invention, is less than 23%.

According to one embodiment of the invention the butter is different from the cacao butter. More specifically the composition of the invention does not contain a cacao butter.

In one preferred variant of the invention, the weight content of $C_{16}$ fatty acid triglycerides, expressed relative to the total amount of fatty acid triglycerides, ranges from 0 to 22%, better still from 0 to 15% and even better still from 2% to 12%.

Preferentially, the butter(s) according to the invention are chosen from murumuru butter, ucuuba butter, shorea butter, illipé butter, shea butter and cupuaçu butter, and even more preferentially from murumuru butter and ucuuba butter.

Composition A according to the invention may comprise one or more butters in an amount inclusively between 1% and 80% by weight, relative to the total weight of the composition, more particularly inclusively between 2% and 50% by weight, preferentially inclusively between 3% and 40% by weight and more preferentially between 5% and 25% by weight.

The waxes may be fatty alcohols or fatty esters that are solid at room temperature and at atmospheric pressure.

According to one particular embodiment of the invention, the composition comprises as third constituent one or more solid fatty alcohols.

The fatty alcohols that are suitable for use in the invention are more particularly chosen from linear saturated alcohols comprising from 6 to 30 carbon atoms and preferably from 8 to 30 carbon atoms. Mention may be made, for example, of cetyl alcohol, stearyl alcohol and their mixture (cetearyl alcohol).

As regards the solid fatty acid esters and/or fatty alcohols, mention may preferably be made of esters of saturated linear fatty acids and of saturated linear fatty alcohols, such as cetyl palmitate, stearyl stearate or cetyl stearate.

According to another particular embodiment of the invention, the composition comprises as third constituent one or more waxes, other than the fatty alcohols and fatty esters mentioned above, preferably of plant origin.

The non-silicone wax(es) are chosen in particular from carnauba wax, candelilla wax, esparto wax, paraffin wax, ozokerite, plant waxes, such as olive tree wax, rice wax, hydrogenated jojoba wax or absolute flower waxes, such as the blackcurrant blossom essential wax sold by Bertin (France), or animal waxes, such as beeswaxes or modified beeswaxes (cerabellina); other waxes or waxy starting materials that can be used according to the invention are in particular marine waxes, such as that sold by Sophim under the reference M82, polyethylene waxes or polyolefin waxes in general.

According to another particular embodiment of the invention, the composition comprises one or more silicone waxes, resins or gums.

In the category of polydialkylsiloxanes, mention may be made of the waxes sold under the names Abil Wax® 9800 and 9801 by the company Goldschmidt, which are polydi($C_1$-$C_{20}$)alkylsiloxanes.

The silicone gums that may be used in accordance with the invention are especially polydialkylsiloxanes and preferably polydimethylsiloxanes with high number-average molecular weights of between 200 000 and 1 000 000, used alone or as a mixture in a solvent. This solvent can be chosen from volatile silicones, polydimethylsiloxane (PDMS) oils, polyphenylmethylsiloxane (PPMS) oils, isoparaffins, polyisobutylenes, methylene chloride, pentane, dodecane or tridecane, or mixtures thereof.

Products that can be used more particularly in accordance with the invention are mixtures such as:

- the mixtures formed from a hydroxy-terminated polydimethylsiloxane or dimethiconol (CTFA), and from a cyclic polydimethylsiloxane, also known as cyclomethicone (CTFA), such as the product Q2 1401 sold by the company Dow Corning;
- mixtures of a polydimethylsiloxane gum and of a cyclic silicone, such as the product SF 1214 Silicone Fluid from the company General Electric; this product is an SF 30 gum corresponding to a dimethicone, having a number-average molecular weight of 500 000, dissolved in the oil SF 1202 Silicone Fluid corresponding to decamethylcyclopentasiloxane;

- mixtures of two PDMSs with different viscosities, and more particularly of a PDMS gum and a PDMS oil, such as the product SF 1236 from the company General Electric. The product SF 1236 is a mixture of a gum SE 30 defined above with a viscosity of 20 $m^2/s$ and of an oil SF 96 with a viscosity of $5\times10^{-6}m^2/s$. This product preferably comprises 15% of gum SE 30 and 85% of an oil SF 96.

The organopolysiloxane resins that can be used in accordance with the invention are crosslinked siloxane systems containing the following units:
$R_2SiO_{2/2}$, $R_3SiO_{1/2}$, $RSiO_{3/2}$ and $SiO_{4/2}$
in which R represents an alkyl containing 1 to 16 carbon atoms. Among these products, the ones that are particularly preferred are those in which R denotes a $C_1$-$C_4$ lower alkyl group, more particularly methyl.

Among these resins, mention may be made of the product sold under the name Dow Corning 593 or those sold under the names Silicone Fluid SS 4230 and SS 4267 by the company General Electric, which are silicones of dimethyl/trimethylsiloxane structure.

Mention may also be made of the trimethyl siloxysilicate type resins sold especially under the names X22-4914, X21-5034 and X21-5037 by the company Shin-Etsu.

Preferably, the fatty substance(s) do not comprise any $C_2$-$C_3$ oxyalkylene units or any glycerol units.

Composition A according to the invention preferably comprises a content of fatty substances other than the oil(s) as defined previously ranging from 0.5% to 50% by weight, better still from 1% to 30% by weight and even better still from 1% to 20% by weight relative to the total weight of the composition.

*The compositions*

Composition A of the invention is cosmetic, i.e. it is cosmetically acceptable and therefore suitable for use for application to keratin fibres.

Preferentially, the composition of the invention does not contain any "mordants", i.e. metal salts conventionally used in "mordanting" (see for example Ullmann's Encyclopedia of Industrial Chemistry, "Textile Dyeing", Herbert Leube et al., DOI: 10.1002/14356007.a26_351, and in particular point 4.8.2, p. 72 ; *ibid,* "Metal-complex dyes", Klaus Gryschtol et al., DOI: 10.1002/14356007.a16_299).

The composition may comprise water or a mixture of water and of one or more organic solvents or a mixture of organic solvents. Composition A according to the invention preferably comprises less than 3% by weight and preferably less than 2% by weight of water relative to the total weight of the composition, or even is free of water. Preferably, the composition according to the invention does not comprise any water other than the water associated with the starting materials included in its composition. It is then referred to as an anhydrous composition.

The composition according to the invention is preferentially in compact form. As emerges from the foregoing, the compact composition according to the invention is "solid".

- "solid" means the state of the composition at room temperature (25°C) and at atmospheric pressure (760 mmHg), i.e. a composition of high consistency, which conserves its form during storage. In contrast to "fluid" compositions, it does not flow under its own weight. It is advantageously characterized by a hardness as defined below.
- "compact composition" means that the composition consists of a mixture of products whose cohesion is at least partly provided by compacting or pressing during the manufacture. In particular, by carrying out a measurement using a TA.XT.plus Texture Analyser sold by Stable Micro Systems, the compact powder according to the invention can advantageously exhibit a resistance to pressure of between 0.2 and 2.5 kg and in particular between 0.8 and 1.5 kg, with respect to the surface area of the spindle used (in the case in point, 7.07 $mm^2$). The measurement of this resistance is performed by moving an SMS P/3 flat-headed cylindrical spindle in contact with the powder over a distance of 1.5 mm and at a speed of 0.5 mm/sec.

According to one preferred embodiment of the invention, composition A is in compact form and in different forms as a function of the desired compacting, especially in the form of pebbles, in the form of stones, in the form of soaps, in the form of pyramids, in the form of bricks or in the form of plates.

The cosmetic composition A of the invention may be in various non-compact galenical forms, such as a lotion, a mousse, a cream or a gel, or in any other form that is suitable for dyeing keratin fibres. It may also be conditioned in a propellant-free pump-action bottle or under pressure in an aerosol container in the presence of a propellant and form a foam.

*Aqueous composition B*

As mentioned previously, another subject of the invention is composition B derived from the mixture between

composition A, which is preferably compact and/or anhydrous, and water.

Preferably, composition B is the form of a poultice.

To do this, composition A according to the invention, preferably in compact and/or anhydrous form comprising the ingredients i), ii) and optionally iii) as defined previously, is mixed with water to obtain a poultice in order to obtain a creamy and pleasant consistency. When the composition is compact, it is crumbled into water. The ratios of composition A according to the invention and water preferably range from 1 part by weight of composition per 1 part by weight of water (1/1) to 1 part by weight of composition A per 3 parts by water (1/3), more preferentially 1 part by weight of composition A per 2 parts of and water (1/2).

According to another particular embodiment of the invention, composition B comprises only ingredients of natural origin.

During the preparation of the poultice, one or more identical or different clays, as defined below, may be added.

According to another preferred embodiment of the invention, composition B is at a neutral pH close to 7 (preferably ranging from 6 to 8 and better still from 6.5 to 7.5).

*Organic solvents:*

Composition A or Bmay comprise one or more organic solvents. Examples of organic solvents that may be mentioned include $C_1$-$C_4$ lower alcohols, such as ethanol and isopropanol; polyols and polyol ethers such as 2-butoxyethanol, propylene glycol, propylene glycol monomethyl ether, diethylene glycol monoethyl ether and monomethyl ether, hexylene glycol, and also aromatic alcohols, for instance benzyl alcohol or phenoxyethanol.

The organic solvents are present in proportions preferably of between 0.1% and 20% by weight approximately and even more preferentially between 0.5% and 10% by weight approximately relative to the total weight of the composition under consideration.

*Adjuvants:*

Compositions A and/or B of the invention may also contain various adjuvants conventionally used in hair dye compositions, such as anionic, cationic, nonionic, amphoteric or zwitterionic surfactants or mixtures thereof, anionic, cationic, nonionic, amphoteric or zwitterionic polymers or mixtures thereof, mineral or organic thickeners, and in particular anionic, cationic, nonionic and amphoteric polymeric associative thickeners, antioxidants, penetrants, sequestrants, fragrances, buffers, dispersants, conditioning agents other than the butters of the invention, for instance ceramides, film-forming agents, preserving agents, opacifiers and mineral or organic thickeners such as clays.

Preferably, compositions A and/or B are not in emulsion form.

Preferably, compositions A and/or B do not contain any surfactants.

The above adjuvants are generally present in an amount for each of them of between 0.01% and 40% by weight relative to the weight of the composition, and preferably between 0.1% and 20% by weight relative to the weight of the composition under consideration.

Needless to say, a person skilled in the art will take care to select this or these optional additional compound(s) such that the advantageous properties intrinsically associated with the composition or the poultice that are useful in the dyeing process in accordance with the invention are not, or are not substantially, adversely affected by the envisaged addition(s).

*The additional dyes:*

Compositions A and/or B of the invention comprising the ingredients i) and ii) and optionally iii) as defined previously may also contain one or more additional direct dyes other than red henna powder i).

These direct dyes are chosen, for example, from those conventionally used in direct dyeing, and among which mention may be made of any commonly used aromatic and/or non-aromatic dye such as neutral, acidic or cationic nitrobenzene direct dyes, neutral, acidic or cationic azo direct dyes, natural direct dyes, neutral, acidic or cationic quinone and in particular anthraquinone direct dyes, azine, triarylmethane, indoamine, methine, styryl, porphyrin, metalloporphyrin, phthalocyanine, cyanine and methine direct dyes, and fluorescent dyes.

Preferentially, the composition or the poultice of the invention comprises one or more natural dyes other than the red henna i) as defined previously. Among the natural direct dyes, mention may be made of juglone, indigo, isatin, curcumin, spinulosin, apigenidin, orceins and lawsone, in defined compound form.

These natural dyes, besides their defined compound form (other than lawsone), may be added in the form of extracts or of plant parts. The said defined compounds from extracts or from plant parts are preferably in the form of powders, in particular fine powders whose particles have sizes identical to that of the red henna powder as defined previously.

The natural or non-natural direct dye(s), other than the red henna i), in the composition according to the invention particularly represent from 0.001 % to 10% by weight relative to the total weight of the composition and even more preferentially from 0.05% to 5% by weight relative to the total weight of the composition under consideration.

Preferably, the composition of the invention does not contain any synthetic direct dyes, i.e. dyes that do not occur in nature.

Compositions A and/or B according to the invention comprising the ingredients i) and ii) as defined previously may also comprise one or more oxidation bases and/or one or more couplers conventionally used for the dyeing of keratin fibres.

Mention may be made, among the oxidation bases, of para-phenylenediamines, bis(phenyl)alkylenediamines, para-aminophenols, bis-para-aminophenols, ortho-aminophenols, heterocyclic bases and their addition salts.

Mention may in particular be made, among these couplers, of meta-phenylenediamines, meta-aminophenols, meta-diphenols, naphthalene couplers, heterocyclic couplers and their addition salts.

The oxidation base(s) present in the composition(s) are each generally present in an amount of between 0.001 % and 10% by weight, of the total weight of the dye composition(s).

Preferably, compositions A and/or B do not contain any oxidation dyes.

*pH of composition B*

According to one particular mode of the invention, the pH of the aqueous composition B containing the ingredients i), ii) and optionally iii) is neutral, i.e. it has a pH of about 7.

According to one particular mode of the invention, composition B of the invention is acidic and preferably has a pH ranging from 3 to 6.5.

The pH of composition B may be adjusted to the desired value by means of acidifying or basifying agents usually used in the dyeing of keratin fibres or alternatively with the aid of standard buffer systems, or of clays as defined previously present in composition A or in the aqueous composition mixed with composition A to give composition B.

Among the acidifying agents for the compositions used in the invention, examples that may be mentioned include mineral or organic acids, for instance hydrochloric acid, orthophosphoric acid or sulfuric acid, carboxylic acids, for instance acetic acid, tartaric acid, citric acid and lactic acid, and sulfonic acids; the acid is preferably an organic acid such as citric acid.

One advantageous variant involves adding a basifying agent to the composition or the poultice according to the invention. More particularly, this alkaline agent is chosen from aqueous ammonia, alkali metal carbonates, alkanolamines such as monoethanolamine, diethanolamine or triethanolamine, and also derivatives thereof, sodium hydroxide, potassium hydroxide and the compounds of formula (I) below:

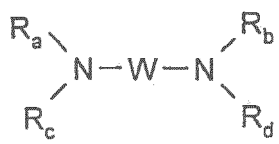

(I)

in which formula (I) W is a propylene residue optionally substituted with a hydroxyl group or a $C_1$-$C_4$ alkyl radical; $R_a$, $R_b$, $R_c$ and $R_d$, which may be identical or different, represent a hydrogen atom or a $C_1$-$C_4$ alkyl or $C_1$-$C_4$ hydroxyalkyl radical.

One variant of the invention concerns the composition or the poultice of the invention that is at a neutral pH.

*Process for preparing the composition of the invention*

The composition of the invention may be obtained in the following manner:
The ingredients i), ii) and optionally iii) as defined are mixed by hand or with a standard mixer and/or an extruder.

*Dyeing process using the composition of the invention*

According to one particular embodiment of the invention, the dyeing process is performed in several steps:

- the first step consists in preparing composition B of the invention, in particular in the form of a creamy poultice, as defined previously, starting with composition A of the invention;
- in the second step, composition B is applied to the keratin fibres and is left on the said fibres preferably for a minimum time of 30 minutes, preferentially a time ranging from 30 minutes to 24 hours and better still ranging from 1 hour to

12 hours;
- in the third step, the keratin fibres are rinsed with water until the poultice has disappeared, preferably without shampooing;
- the keratin fibres may then be dried or left to dry naturally, without a hairdryer.

According to another particular embodiment of the invention, the dyeing process is performed in several steps:

- the first step consists in preparing composition B of the invention as described previously;
- in the second step, composition B is left to stand for several hours, preferably 24 hours, and composition B is then applied and left on the said fibres preferably for a minimum time of 30 minutes (preferably ranging from 30 minutes to 24 hours and better still from 1 hour to 12 hours);

- in the third step, the keratin fibres are rinsed with water until the poultice has disappeared, preferably without shampooing;
- the keratin fibres may then be dried or left to dry naturally, without a hairdryer.

The water mixed with composition A, used in the first step may be at room temperature or at a higher temperature, in particular at a temperature ranging from 40°C to 98°C.
According to another embodiment of the invention, the composition is mixed with or crumbled into water, at a temperature below 40°C, in particular between 10°C and 40°C.
Preferably, the ratio of the amount by weight of composition A of the invention/amount by weight of water ranges from 1/1 to 1/3 and is preferably 1/2.
According to a particularly advantageous process, after the third step, the keratin fibres are:

a) either mechanically wiped with a towel or absorbent paper,
b) or dried by heat with a heat source (convection, conduction or radiation) by passing over, for example, a stream of a warm gas such as air necessary to evaporate off the solvent(s); heat sources that may be mentioned include a hairdryer, hairdrying hoods, a hair-straightening iron, an infrared ray dispenser and other standard heating appliances.

Irrespective of the application method, the application temperature for composition B ranges from room temperature (15 to 25°C) to 80°C and more particularly from 15 to 45°C. Thus, after application of the poultice according to the invention, the head of hair may advantageously be subjected to a heat treatment by heating to a temperature ranging from 30 to 60°C. In practice, this operation may be performed using a styling hood, a hairdryer, an infrared ray dispenser or other standard heating appliances.
Use may be made, both as means for heating and straightening the hair, of a heating iron at a temperature ranging from 60°C to 220°C and preferably from 120°C to 200°C.
A specific form of the invention relates to a dyeing method which is carried out at ambient temperature (25°C).

## I) EXAMPLES OF DYEING

The following compositions were prepared:
The percentages are indicated on a weight basis relative to 100 g of composition.

**Composition A:**

| | |
|---|---|
| Henna (*Lawsonia inermis*) leaf powder | 78 g% |
| Refined coconut oil | 22 g% |

**Composition A':**

| | |
|---|---|
| Henna (*Lawsonia inermis*) leaf powder | 75 g% |
| Refined coconut oil | 25 g% |

**Composition B**

| | |
|---|---|
| Henna (*Lawsonia inermis*) leaf powder | 70 g% |
| Refined murumuru butter | 5 g% |
| Refined coconut oil | 25 g% |

**Composition C**

| | |
|---|---|
| Henna (*Lawsonia inermis*) leaf powder | 75 g% |
| Refined coconut oil | 12 g% |
| Refined sunflower oil | 13 g% |

Compositions **A, A', B,** and **C** were compacted in a conventional manner. The compositions of the invention, even compacted, are easy to split by hand, while at the same time not being pulverulent. 1 part of one of the three compositions **A, A', B,** or **C** is mixed with 2 parts of warm water at 37 °C in a bowl, or in cold water (10 °C).

The mixing is performed easily, and the compositions, even compacted, break down rapidly in water. The poultice obtained is very creamy, and is easy to apply to the keratin fibres, totally impregnating the keratin fibres from the root to the end.

The poultice is applied to dry natural grey hair containing 90% white hairs, with a leave-on time of 60 minutes.

The hair is rinsed thoroughly.

The hair is dried.

A strong coppery aesthetic coloration is obtained especially very intense and/or chromatic. The hair is soft and smooth, and the coloration is very uniform from one fibre to another and from the root to the end.

**Colorimetric results:**

The coloring of the hair is evaluated visually and read on a Minolta spectrocolorimeter (CM3600d, illuminant D65, angle 10°, SCI values) for the L*, a*, b* colorimetric measurements.

In this L*, a*, b* system, L* represents the intensity of the color, a* indicates the green/red color axis and b* indicates the blue/yellow color axis. The lower the value of L, the darker or more intense the color. The higher the value of a*, the redder the shade; the higher the value of b*, the yellower the shade.

The variation in coloring between the colored locks of natural white hair which is untreated (control) and after treatment or coloration are defined by ΔE*, corresponding to the colour uptake on keratin fibers, according to the following equation:

$$\Delta E^* = \sqrt{(L^* - L_o^*)^2 + (a^* - a_o^*)^2 + (b^* - b_o^*)^2}$$

In this equation, L*, a* and b* represent the values measured after dyeing the natural hair comprising 90% of white hairs and $L_0$*, $a_0$* and $b_0$* represent the values measured for the untreated natural hair comprising 90% of white hairs.

The greater the value of ΔE, the greater the difference in color between the control locks and the dyed locks and the greater colour uptake is.

**Chromaticity: C***

Chromaticity in the CIE L*, a*, b* colorimetric system is calculated according to the following equation :

$$C^* = \sqrt{a^{*2} + b^{*2}}$$

The greater the value of C*, the greater the chromaticity is.

Table: Results of dyeing vs. untreated lock

| | L* | a* | b* | C* | ΔL* | ΔE* |
|---|---|---|---|---|---|---|
| Untreated 90 % white natural hair | 65,56 | 0,67 | 13,82 | 13,84 | ------ | ------ |
| Hair after coloration with A’ | 51,48 | 12,11 | 27,04 | 29,63 | -14,08 | 22,45 |

It is apparent from the above table that the locks of natural white hair treated with the composition according to the invention make it possible to dye in a significantly intense and chromatic way.

**Claims**

1. Cosmetic composition A, in compact or non compact form, comprising:

   i) at least 20% by weight, relative to the weight of the composition, of red henna powder, preferably as fine particles, and
   ii) at least one oil chosen from babassu oil, sunflower oil, olive oil, coconut oil, Brazil nut oil, marula oil, corn oil, argan oil, soybean oil, grapeseed oil, flax oil, sesame oil, hazelnut oil, apricot oil, macadamia oil, arara oil, castor oil, avocado oil, shea butter oil, coprah oil and rapeseed oil;

   in which the oil(s) are in an amount inclusively between 1% and 80% by weight relative to the total weight of the composition.

2. Composition according to the preceding claim, in compact and/or anhydrous form.

3. Composition according to either of Claims 1 and 2, in which the henna powder consists of fine particles less than or equal to 500 μm in size; preferentially, the powder consists of fine particles with sizes inclusively between 50 and 300 μm and more particularly between 10 and 200 μm.

4. Composition according to any one of the preceding claims, which also comprises one or more fatty substances, other than the oil(s), the said additional fatty substance(s) preferably being chosen from butters.

5. Composition according to the preceding claim, in which the butter(s) are of plant origin, and are more particularly chosen from shea butter, Karité Nilotica butter (*Butyrospermum parkii*), galam butter, *(Butyrospermum parkii),* Borneo butter or fat or tengkawang tallow (*Shorea stenoptera*), shorea butter, illipé butter, madhuca butter or *Bassia madhuca longifolia* butter, mowrah butter (*Madhuca latifolia*), katiau butter (*Madhuca mottleyana*), phulwara butter (*M. butyracea*), mango butter (*Mangifera indica*), murumuru butter *(Astrocaryum murumuru*), kokum butter (*Garcinia indica*), ucuuba butter (*Virola sebifera*), tucuma butter, painya butter (Kpangnan) (*Pentadesma butyracea*), coffee butter *(Coffea arabica),* apricot butter (*Prunus armeniaca*), macadamia butter (*Macadamia ternifolia*), grapeseed butter (*Vitis vinifera*), avocado butter *(Persea gratissima),* olive butter *(Olea europaea),* sweet almond butter *(Prunus amygdalus dulcis),* cocoa butter (*Theobroma* cacao) and sunflower butter.

6. Composition according to Claim 4 or 5, which comprises one or more identical or different butters, in which the weight content of $C_{16}$ fatty acid triglycerides, expressed relative to the total content of fatty acid triglycerides, is less than 23%.

7. Composition according to any one of Claims 4 to 6, in which the butter(s) are chosen from murumuru butter, ucuuba butter, shorea butter, illipé butter and shea butter, and even more preferentially from murumuru butter and ucuuba butter.

8. Composition according to any one of the preceding claims, in which the red henna powder is in an amount inclusively between 20% and 99% by weight relative to the total weight of the composition, particularly between 30% and 95% relative to the total weight of the composition, more particularly between 40% and 90% relative to the total weight of the composition, preferentially between 50% and 85% and more preferentially between 60% and 80% relative to the total weight of the composition.

9. Composition according to any one of the preceding claims, in which the oil(s) are in an amount inclusively between

2% and 50% relative to the total weight of the composition, preferentially between 3% and 40% relative to the total weight of the composition and more preferentially between 5% and 25% relative to the total weight of the composition.

**10.** Composition according to any one of the preceding claims, which comprises one or more fatty substances other than the oil(s), in a total content ranging from 0.5% to 50% by weight, better still from 1% to 30% by weight and even better still from 1% to 20% by weight relative to the total weight of the composition.

**11.** Composition according to any one of the preceding claims, in which the composition consists only of ingredients of natural origin.

**12.** Composition according to any one of the preceding claims, which is in compact form and especially in the form of pebbles, in the form of stones, in the form of soaps, in the form of pyramids, in the form of bricks or in the form of plates.

**13.** Aqueous composition B prepared from a mixture of a composition according to any one of the preceding claims and water, preferably in proportions ranging from 1 part by weight of composition according to any one of the preceding claims per 1 part by weight of water (1/1) to 1 part by weight of composition according to any one of the preceding claims per 3 parts by weight of water (1/3), more preferentially 1 part by weight of composition according to any one of the preceding claims per 2 parts by weight of water by weight (1/2); composition B is particularly in the form of a poultice.

**14.** Composition B according to the preceding claim, which has a neutral pH.

**15.** Process for dyeing keratin fibres using the following steps:

- in the first step, the preparation of a composition B according to Claim 13 or 14;
- in the second step, composition B is:

a) either immediately applied to the keratin fibres, and left on the fibres for a minimum time of 30 minutes, preferentially ranging from 30 minutes to 24 hours and better still from 1 hour to 12 hours,
b) or left to stand for several hours, preferably 24 hours, and then applied and left on the fibres for a minimum time of 30 minutes, preferably ranging from 30 minutes to 24 hours and better still from 1 hour to 12 hours;

- in the third step, the keratin fibres are rinsed with water until the poultice has disappeared, preferably without shampooing;
- the keratin fibres may then be dried with a source of heat or left to dry naturally at room temperature.

**16.** Use of composition A according to any one of Claims 1 to 12 or of composition B according to Claim 13 or 14, for dyeing keratin fibres such as the hair.

## Patentansprüche

**1.** Kosmetikzusammensetzung A in kompakter oder nichtkompakter Form, umfassend:

i) mindestens 20 Gew.%, bezogen auf das Gewicht der Zusammensetzung, von rotem Hennapulver, vorzugsweise als feine Partikel, und
ii) mindestens ein Öl ausgewählt aus Babassu-Öl, Sonnenblumenöl, Olivenöl, Kokosnussöl, Paranussöl, Marulaöl, Maisöl, Arganöl, Sojaöl, Traubenkernöl, Flachsöl, Sesamöl, Haselnussöl, Aprikosenöl, Macadamiaöl, Araraöl, Castoröl, Avocadoöl, Sheabutter-Öl, Kopraöl und Rapsöl;

wobei das Öl bzw. die Öle in einer Menge zwischen 1 Gew.% und 80 Gew.% einschließlich vorhanden sind, bezogen auf das Gesamtgewicht der Zusammensetzung.

**2.** Zusammensetzung nach einem der vorhergehenden Ansprüche in kompakter und/oder wasserfreier Form.

**3.** Zusammensetzung nach einem der Ansprüche 1 und 2, wobei das Hennapulver aus feinen Partikeln in einer Größe kleiner als oder gleich 500 $\mu$m besteht, wobei das Pulver vorzugsweise aus feinen Partikeln mit Größen zwischen 50 und 300 $\mu$m einschließlich und insbesondere zwischen 10 und 200 $\mu$mbesteht.

**4.** Zusammensetzung nach einem der vorhergehenden Ansprüche, die auch ein oder mehrere Fettsubstanzen umfasst, die von dem Öl bzw. den Ölen verschieden sind, wobei die zusätzliche(n) Fettsubstanz(en) vorzugsweise ausgewählt ist bzw. sind aus Butterarten.

**5.** Zusammensetzung nach einem der vorhergehenden Ansprüche, wobei die Butterart(en) pflanzlicher Herkunft ist bzw. sind und insbesondere ausgewählt sind aus Sheabutter, Karite Nilotica-Butter *(Butyrospermum parkii),* Galambutter, *(Butyrospermum parkii),* Borneobutter oder -fett öder Tengkawang-Talg (*Shorea stenoptera*), Shoreabutter, Illipé-Butter, Madhuca-Butter oder *Bassia madhuca longifolia*-Butter, Mowrah-Butter (*Madhuca latifolia*), Katiau-Butter (*Madhuca mottleyana*), Phulwarabutter *(M. butyracea)*, Mangobutter *(Mangifera indica),* Murumuru-Butter *(Astrocaryum murumuru),* Kokumbutter *(Garcinia indica),* Ucuuba-Butter *(Virola sebifera),* Tucuma-Butter, Painya-Butter (Kpangnan) *(Pentadesma butyracea),* Kaffeebutter *(Coffea arabica),* Aprikosenbutter *(Prunus armeniaca),* Macadamiabutter *(Macadamia ternifolia),* Traubenkernbutter *(vitis vinifera),* Avocadobutter *(Persea gratissima),* Olivenbutter *(Olea europaea),* Süßmandelbutter *(Prunus amygdalus dulcis)*, Kakaobutter *(Theobroma cacao)* und Sonnenblumenbutter.

**6.** Zusammensetzung nach Anspruch 4 oder 5, die ein oder mehr identische oder unterschiedliche Butterarten umfasst, wobei der Gewichtgehalt an Triglyceriden von $C_{16}$-Fettsäurent, ausgedrückt bezogen auf den Gesamtgehalt an Fettsäuretriclyceriden, kleiner als 23 % ist.

**7.** Zusammensetzung nach einem der Ansprüche 4 bis 6, wobei die Butterart(en) ausgewählt ist bzw. sind aus Murumuru-Butter, Ucuuba-Butter, Shoreabutter, Illipe-Butter und Sheabutter und noch bevorzugter aus Murumuru-Butter und Ucuuba-Butter.

**8.** Zusammensetzung nach einem der vorhergehenden Ansprüche, wobei das rote Hennapulver in einer Menge zwischen 20 Gew.% und 99 Gew.% einschließlich vorhanden ist, bezogen auf das Gesamtgewicht der Zusammensetzung, insbesondere zwischen 30 % und 95 %, bezogen auf das Gesamtgewicht der Zusammensetzung, besonders zwischen 40 % und 90 %, bezogen auf das Gesamtgewicht der Zusammensetzung, vorzugsweise zwischen 50 % und 85 % und bevorzugter zwischen 60 % und 80 %, bezogen auf das Gesamtgewicht der Zusammensetzung.

**9.** Zusammensetzung nach einem der vorhergehenden Ansprüche, wobei das Öl bzw. die Öle in einer Menge zwischen 2 % und 50 % einschließlich, bezogen auf das Gesamtgewicht der Zusammensetzung, vorzugsweise zwischen 3 % und 40 %, bezogen auf das Gesamtgewicht der Zusammensetzung und bevorzugter zwischen 5 % und 25%, bezogen auf das Gesamtgewicht der Zusammensetzung, vorhanden ist bzw. sind.

**10.** Zusammensetzung nach einem der vorhergehenden Ansprüche, die eine oder mehrere von dem Öl bzw. den Ölen verschiedene Fettsubstanzen in einem Gesamtgehalt im Bereich von 0,5 Gew.% bis 50 Gew.% umfasst, besser noch 1 Gew.% bis 30 Gew.% und sogar noch besser 1 Gew.% bis 20 Gew.%, bezogen auf das Gesamtgewicht der Zusammensetzung.

**11.** Zusammensetzung nach einem der vorhergehenden Ansprüche, wobei die Zusammensetzung nur aus Bestandteilen natürlicher Herkunft besteht.

**12.** Zusammensetzung nach einem der vorhergehenden Ansprüche, die in kompakter Form und insbesondere in Form von Kieseln, in Form von Steinen, in Form von Seifen, in Form von Pyramiden, in Form von Ziegeln oder in Form von Platten vorliegt.

**13.** Wässrige Zusammensetzung B, die aus einer Mischung einer Zusammensetzung gemäß einem der vorhergehenden Ansprüche und Wasser hergestellt ist, vorzugsweise in Anteilen im Bereich von 1 Gewichtsteil der Zusammensetzung gemäß einem der vorhergehenden Ansprüche auf 1 Gewichtsteil Wasser (1/1) bis 1 Gewichtsteil der Zusammensetzung gemäß einem der vorhergehenden Ansprüche auf 3 Gewichtsteile Wasser (1/3), bevorzugter 1 Gewichtsteil der Zusammensetzung gemäß einem der vorhergehenden Ansprüche auf 2 Gewichtsteile Wasser nach Gewicht (1/2); wobei Zusammensetzung B insbesondere in Form einer Packung vorliegt.

**14.** Zusammensetzung B nach dem vorhergehenden Anspruch, die einen neutralen pH-Wert aufweist.

**15.** Verfahren zum Färben von Keratinfasern unter Verwendung der folgenden Schritte:

- in dem ersten Schritt Herstellen einer Zusammensetzung B nach Anspruch 13 oder 14;

- in dem zweiten Schritt wird Zusammensetzung B

a) entweder sofort auf die Keratinfasern aufgetragen und für eine Mindestzeit von 30 Minuten, vorzugsweise im Bereich von 30 Minuten bis 24 Stunden und besser noch von 1 Stunde bis 12 Stunden auf den Fasern belassen,
b) oder mehrere Stunden, vorzugsweise 24 Stunden stehen gelassen und danach auf die Fasern aufgetragen und für eine Mindestzeit von 30 Minuten, vorzugsweise im Bereich von 30 Minuten bis 24 Stunden und besser noch von 1 Stunde bis 12 Stunden auf den Fasern belassen;

- in dem dritten Schritt werden die Keratinfasern mit Wasser gespült, bis die Packung verschwunden ist, vorzugsweise ohne Schamponieren;
- wobei die Keratinfasern dann mit einer Wärmequelle getrocknet oder natürlicherweise bei Raumtemperatur trocknen gelassen werden können.

**16.** Verwendung von Zusammensetzung A nach einem der Ansprüche 1 bis 12 oder der Zusammensetzung B nach Anspruch 13 oder 14 zum Färben von Keratinfasern, wie den Haaren.

**Revendications**

**1.** Composition cosmétique A, sous forme compacte ou non compacte, comprenant :

i) au moins 20% en poids, par rapport au poids de la composition, de poudre de henné rouge, préférablement sous forme de particules fines, et
ii) au moins une huile choisie parmi l'huile de babassu, l'huile de tournesol, l'huile d'olive, l'huile de noix de coco, l'huile de noix du Brésil, l'huile de marula, l'huile de maïs, l'huile d'argan, l'huile de soja, l'huile de pépins de raisin, l'huile de lin, l'huile de sésame, l'huile de noisette, l'huile d'abricot, l'huile de macadamia, l'huile d'arara, l'huile de ricin, l'huile d'avocat, l'huile de beurre de karité, l'huile de coprah, et l'huile de colza ;

dans laquelle la ou les huiles se trouvent selon une quantité comprise de manière inclusive entre 1% et 80% en poids par rapport au poids total de la composition.

**2.** Composition selon la revendication précédente, sous forme compacte et/ou anhydre.

**3.** Composition selon la revendication 1 ou bien 2, dans laquelle la poudre de henné est constituée de particules fines d'une taille inférieure ou égale à 500 $\mu$m ; préférablement, la poudre est constituée de particules fines de tailles comprises de manière inclusive entre 50 et 300 $\mu$m et plus particulièrement entre 10 et 200 $\mu$m.

**4.** Composition selon l'une quelconque des revendications précédentes, qui comprend également une ou plusieurs substances grasses, autres que la ou les huiles, ladite ou lesdites substances grasses supplémentaires étant choisies de préférence parmi des beurres.

**5.** Composition selon la revendication précédente, dans laquelle le ou les beurres sont d'origine végétale, et sont choisis de préférence parmi le beurre de karité, le beurre de karité Nilotica *(Butyrospermum parkii),* le beurre de Galam, *(Butyrospermum parkii),* le beurre ou la graisse de Bornéo ou suif de tengkawang (*Shorea stenoptera*), le beurre de shorea, le beurre d'illipé, le beurre de madhuca ou le beurre de *Bassia* ou *Madhuca longifolia*, le beurre de mowrah (*Madhuca latifolia*), le beurre de katiau *(Madhuca mottleyana)*, le beurre de phulwara (*M. butyracea*), le beurre de mangue (*Mangifera indica*), le beurre de murumuru (*Astrocaryum murumuru*), le beurre de kokum (*Garcinia indica*), le beurre d'ucuuba (*Virola sebifera*), le beurre de tucuma, le beurre de painya (Kpangnan) (*Pentadesma butyracea*), le beurre de café (*Coffea arabica),* le beurre d'abricot (*Prunus armeniaca*), le beurre de macadamia (*Macadamia ternifolia*), le beurre de pépins de raisin (*Vitis vinifera*), le beurre d'avocat *(Persea gratissima),* le beurre d'olive (*Olea europaea),* le beurre d'amande douce *(Prunus amygdalus dulcis),* le beurre de cacao (*Theobroma cacao*) et le beurre de tournesol.

**6.** Composition selon la revendication 4 ou 5, qui comprend un ou plusieurs beurres identiques ou différents, où la teneur en poids en triglycérides d'acides gras en $C_{16}$, exprimée par rapport à la teneur totale en triglycérides d'acides gras, est inférieure à 23%.

**7.** Composition selon l'une quelconque des revendications 4 à 6, dans laquelle le ou les beurres sont choisis parmi le beurre de murumuru, le beurre d'ucuuba, le beurre de shorea, le beurre d'illipé et le beurre de karité, et encore plus préférablement parmi le beurre de murumuru et le beurre d'ucuuba.

**8.** Composition selon l'une quelconque des revendications précédentes, dans laquelle la poudre de henné rouge se trouve selon une quantité comprise de manière inclusive entre 20% et 99% en poids par rapport au poids total de la composition, en particulier entre 30% et 95% par rapport au poids total de la composition, plus particulièrement entre 40% et 90% par rapport au poids total de la composition, préférablement entre 50% et 85% et plus préférablement entre 60% et 80% par rapport au poids total de la composition.

**9.** Composition selon l'une quelconque des revendications précédentes, dans laquelle la ou les huiles se trouvent selon une quantité comprise de manière inclusive entre 2% et 50% par rapport au poids total de la composition, préférablement entre 3% et 40% par rapport au poids total de la composition, et plus préférablement entre 5% et 25% par rapport au poids total de la composition.

**10.** Composition selon l'une quelconque des revendications précédentes, qui comprend une ou plusieurs substances grasses autres que la ou les huiles, selon une teneur totale allant de 0,5% à 50% en poids, mieux encore de 1% à 30% en poids et encore mieux de 1% à 20% en poids par rapport au poids total de la composition.

**11.** Composition selon l'une quelconque des revendications précédentes, **caractérisée en ce que** la composition n'est constituée que d'ingrédients d'origine naturelle.

**12.** Composition selon l'une quelconque des revendications précédentes, qui se trouve sous forme compacte et notamment sous forme de galets, sous forme de palets, sous forme de savons, sous forme de pyramides, sous forme de briques, ou sous forme de plaquettes.

**13.** Composition aqueuse B préparée à partir d'un mélange d'une composition selon l'une quelconque des revendications précédentes et d'eau, préférablement selon des proportions allant de 1 partie en poids de composition selon l'une quelconque des revendications précédentes pour 1 partie en poids d'eau (1/1) à 1 partie en poids de composition selon l'une quelconque des revendications précédentes pour 3 parties en poids d'eau (1/3), plus préférablement de 1 partie en poids de composition selon l'une quelconque des revendications précédentes pour 2 parties en poids d'eau en poids (1/2) ; la composition B se trouvant en particulier sous la forme d'un cataplasme.

**14.** Composition B selon la revendication précédente, qui possède un pH neutre.

**15.** Procédé de coloration de fibres kératiniques, utilisant les étapes suivantes :

- dans une première étape, la préparation d'une composition B selon la revendication 13 ou 14 ; dans une deuxième étape, la composition B est

  a) soit appliquée immédiatement aux fibres kératiniques, et laissée sur les fibres pendant un temps minimum de 30 minutes, allant de préférence de 30 minutes à 24 heures et mieux encore de 1 heure à 12 heures ;
  b) soit laissée à reposer pendant plusieurs heures, préférablement 24 heures, puis appliquée et laissée sur les fibres pendant un temps minimum de 30 minutes, allant de préférence de 30 minutes à 24 heures et mieux encore de 1 heure à 12 heures ;

- dans une troisième étape, les fibres kératiniques sont rincées par de l'eau jusqu'à la disparition du cataplasme, préférablement sans shampooiner ;
- les fibres kératiniques peuvent ensuite être séchées par une source de chaleur ou laissées à sécher naturellement à température ambiante.

**16.** Utilisation de la composition A selon l'une quelconque des revendications 1 à 12 ou de la composition B selon la revendication 13 ou 14, pour la coloration de fibres kératiniques telles que les cheveux.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 201003133362 A
- DE 29902432
- DE 19905707
- EP 0806199 A
- JP 2010001278 A
- WO 9739724 A


### Non-patent literature cited in the description


- Dyes, Natural. Kirk-Othmer Encyclopedia of Chemical Technology
- Henna. Encyclopedia Britannica
- Hair preparations. Kirk-Othmer Encyclopedia of Chemical Technology. John Wiley & Sons, Inc,
- **C. BOUILLON ; J. WILKINSON.** The Science of Hair Care. CRC Press, Taylor & Francis Group, 2005, 236-241
- Ullmann's Encyclopedia. Wiley-VCH Verlag GmbH & Co. KGaA, 2006
- **J. SCIBILIA ; E. GALDI ; G. BISCALDI ; G. MOSCATO.** *Allergy,* 1997, vol. 52, 231-232
- Fats and Fatty Oils. **A. THOMAS.** Ullmann's Encyclopedia of Industrial Chemistry
- Textile Dyeing. **HERBERT LEUBE et al.** Ullmann's Encyclopedia of Industrial Chemistry
- Metal-complex dyes. **KLAUS GRYSCHTOL et al.** ULLMAN'S ENCYCLOPEDIA OF INDUSTRIAL CHEMISTRY